(19) Europäisches Patentamt
European Patent Office
Office européen des brevets

(11) **EP 4 717 162 A1**

(12) **EUROPEAN PATENT APPLICATION**

(43) Date of publication:
**01.04.2026  Bulletin 2026/14**

(21) Application number: **25203449.1**

(22) Date of filing: **19.09.2025**

(51) International Patent Classification (IPC):
**A61B 5/08** (2006.01)    **A61B 5/087** (2006.01)
**A61M 16/00** (2006.01)    **A61B 5/00** (2006.01)

(52) Cooperative Patent Classification (CPC):
**A61B 5/0803; A61B 5/087; A61B 5/7278;**
**A61M 16/024;** A61M 2016/0021; A61M 2016/0027;
A61M 2202/0208

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **25.09.2024  US 202418896846**

(71) Applicant: **Dynasthetics, LLC**
**Salt Lake City, UT 84119 (US)**

(72) Inventors:
• **Orr, Joseph**
  **Park City (US)**
• **Blackwell, Trey**
  **Lehi (US)**

(74) Representative: **Gleiss Große Schrell und Partner**
**mbB**
**Leitzstraße 45**
**70469 Stuttgart (DE)**

(54) **SYSTEM TO MONITOR RESPIRATION DURING SEDATION WITH NASAL PRESSURE AND OXYGEN FLOW**

(57)    This disclosure relates to systems and methods for monitoring sedated patients that are receiving supplemental oxygen flows. A method of monitoring a patient's breathing while sedated includes identifying an end expiratory pause in the patient's breathing pattern and obtaining a first respiratory signal corresponding to a nasal pressure of a patient. The method may also include delivering a pulse of high-flow oxygen during the end expiratory pause at a rate of about 30 liters-per-minute (L/min) and delivering the pulse of high-flow oxygen during the end expiratory pause for less than 0.5 seconds. The method may further include measuring a peak nasal pressure and a peak oxygen flow and synthesizing a second respiratory signal. The second respiratory signal may correspond to the peak nasal pressure and the peak oxygen flow, and may be normalized for the patient. The method may additionally include determining the patient's breathing pattern, rate, or frequency.

FIG. 2

**Description**

TECHNICAL FIELD

This disclosure relates generally to systems and methods for monitoring, through nasal pressure and auditory feedback, sedated patients that are receiving supplemental oxygen flows.

SUMMARY

Disclosed are systems, devices, and/or methods of use thereof regarding monitoring sedated patients through nasal pressure and auditory feedback. In various aspects, a method of monitoring a patient's breathing while sedated includes identifying an end expiratory pause in the patient's breathing pattern and obtaining a first respiratory signal corresponding to a nasal pressure of a patient. The method may also include delivering a pulse of high-flow oxygen during the end expiratory pause at a rate of about 30 liters-per-minute (L/min) and delivering the pulse of high-flow oxygen during the end expiratory pause for less than 0.5 seconds. The method may further include measuring a peak nasal pressure and a peak oxygen flow and synthesizing a second respiratory signal. The second respiratory signal may correspond to the peak nasal pressure and the peak oxygen flow, and may be normalized for the patient. The method may additionally include determining the patient's breathing pattern, rate, or frequency.

In various aspects a method of determining whether an airway of a sedated patient is obstructed includes identifying an end expiratory pause in the patient's breathing pattern and obtaining a first respiratory signal corresponding to a nasal pressure of a patient. The method may also include delivering a pulse of high-flow oxygen during the end expiratory pause at a rate of about 30 liters-per-minute (L/min) and delivering the pulse of high-flow oxygen during the end expiratory pause for less than 0.5 seconds. The method may further include measuring a peak nasal pressure and a peak oxygen flow and synthesizing a second respiratory signal. The second respiratory signal may correspond to the peak nasal pressure and the peak oxygen flow, and may be normalized for the patient. The method may additionally include providing auditory feedback indicating whether the airway of the patient is obstructed.

In various aspects, a system for applying a patient-specific calibration in real-time to a nasal pressure or nasal flow waveform includes a nasal cannula having an oxygen inlet tube and a pressure-sensing tube separate from the oxygen inlet tube. The system may also include a processor-actuated valve for controlling oxygen flow from a high-pressure oxygen source to the nasal cannula. Additionally, the system may include a flow sensor in connection with the oxygen inlet tube and for monitoring a flow of oxygen through the processor-actuated valve. Further, the system may include a pressure sensor in connection with the pressure-sensing tube and a processor. The processor may receive one or more pressure signals from the pressure sensor and may be in communication with the processor-actuated valve. The processor may be programmed to actuate the processor-actuated valve upon determination of an end expiratory pause in a breathing pattern of the patient.

In various aspects, a system for applying a patient-specific calibration in real-time to a nasal pressure or nasal flow waveform includes a processor and a display in communication with the processor. The processor may be programmed to receive one or more first respiratory signals corresponding to a nasal pressure of a patient; calculate a calibration factor unique to the patient; receive one or more signals corresponding to a flow of oxygen to the patient; and synthesize a second respiratory signal based on the one or more first respiratory signals, the flow of oxygen to the patient, and the calibration factor. The display may be for displaying the one or more first respiratory signals corresponding to a nasal pressure of a patient; the synthesized second respiratory signal corresponding to the first respiratory signals, the flow of oxygen to the patient, and the calibration factor; a first visual indicator corresponding to the flow of oxygen to the patient and a second visual indicator corresponding to an obstruction in an airway of the patient.

Other aspects of the disclosed subject matter, as well as features and advantages of various aspects of the disclosed subject matter, should be apparent to those of ordinary skill in the art through consideration of the ensuing description, the accompanying drawings, and the appended claims.

BRIEF DESCRIPTION OF THE DRAWINGS

In the drawings:

FIG. 1A illustrates a sample nasal pressure waveform for two breaths for an exemplary unsedated patient and FIG. 1B illustrates the nasal pressure for two breaths for an exemplary sedate patient;
FIG. 2 schematically illustrates one embodiment of a system for monitoring a sedated patient, using nasal pressure and auditory feedback, and calibrating a nasal pressure signal for the sedated patient;
FIG. 3 schematically illustrates another embodiment of a system for monitoring a sedated patient, using nasal pressure and auditory feedback, and calibrating a nasal pressure signal for the sedated patient;

FIG. 4 illustrates how an airway obstruction appears in the calibrated nasal pressure waveform; and
FIGS. 5 and 6 are flowcharts of example methods of monitoring a patient.

DETAILED DESCRIPTION

During medical procedures when a patient is sedated, the patient maintains the ability to breathe on their own, but the drugs used to administer sedation suppress the autonomic respiratory drive such that respiration may become less frequent and more shallow. Supplemental oxygen is therefore given, typically through a nasal cannula or oxygen mask. However, this oxygen is only beneficial if it can reach the patient's lungs. Therefore, it is important for the practitioner to be able to monitor the patient's respiration to ensure adequate oxygen intake. The current options for monitoring respiration in sedated patients are limited.

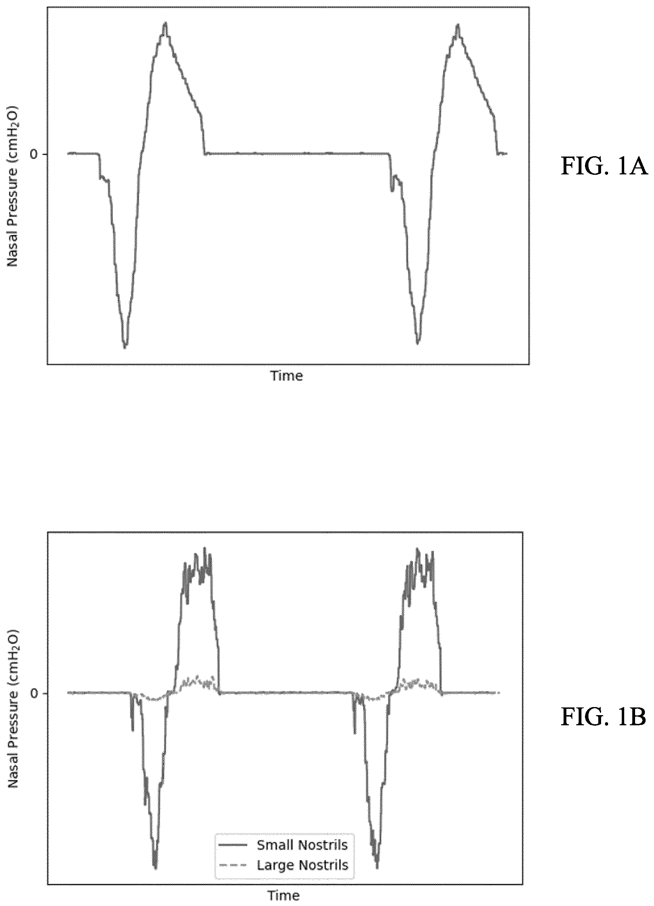

A relatively unexplored biological signal that could be useful for monitoring respiration is the pressure or flow of gas in the nose and/or mouth (nasal pressure). When the patient inhales, nasal pressure drops relative to ambient pressure, thus forcing air and oxygen intake to the lungs. When the patient exhales, nasal pressure rises relative to ambient pressure, which forces expired gas out of the lungs. As shown in FIG. 1A, plotting the nasal pressure signal (y-axis) over time (x-axis) can create an identifiable waveform to aid in monitoring frequency and depth of respiration.

Monitoring airway pressure and flow is straightforward in cases where the patient is endotracheally intubated and their airway is completely secured in a closed system, where all flow and pressure is accounted for by a mechanical ventilator. However, during sedation, the patient's airway is not closed or controlled. Thus, pressure and flow in the airway becomes difficult to measure, and the amplitude of the measured signal may vary dependent on the individual patient anatomy. The amplitude of the nasal pressure signal is a function of the respiratory flow and the resistance to flow between the pressure measurement point (tip of the nasal cannula) and ambient pressure.

For example, as illustrated in FIG. 1B, nasal pressure in a sedated patient (and particularly for a patient with large nostrils) will be much smaller in amplitude than the nasal pressure in a patient with small nostrils taking a breath of the same size. Variables that change the amplitude include size of the breaths (usually larger in unsedated patients), better nostril fit, and if the mouth is open or closed. The signal is stronger when the mouth is closed and all ventilation is through the nose.

Furthermore, if pressure is being measured in the nose, the signal would be much larger in amplitude if the patient were breathing exclusively through their nose, but would be much smaller in amplitude if breathing through both the nose and the mouth. It therefore can become very difficult to utilize the nasal pressure signal, especially as the signal gets very small in amplitude, such as illustrated in FIG. 1B.

The same effect shown in FIG. 1B is observed in a patient wearing an oxygen mask except the difference in signal size is due to the quality of the seal between the mask and the face. When the seal is poor, the observed pressure signal is small. Furthermore, administering oxygen flow creates positive pressure in the nose and/or mouth, which can obscure the amplitude of the nasal pressure signal. This is especially apparent during inhalation, when the positive pressure created by giving oxygen counteracts the negative pressure that indicates the patient is inhaling. As oxygen flows are raised, the problem is exacerbated and may result in no detectable negative pressure during inhalation.

Accordingly, it is beneficial to normalize or calibrate the nasal pressure waveforms for a given patient, where such calibration will also account for the supplemental oxygen flow being delivered to the patient. This will produce a patient-specific signal that can be used to monitor the patient's breathing pattern (e.g., rate, frequency, etc.) and detect airway obstructions in the sedated patient.

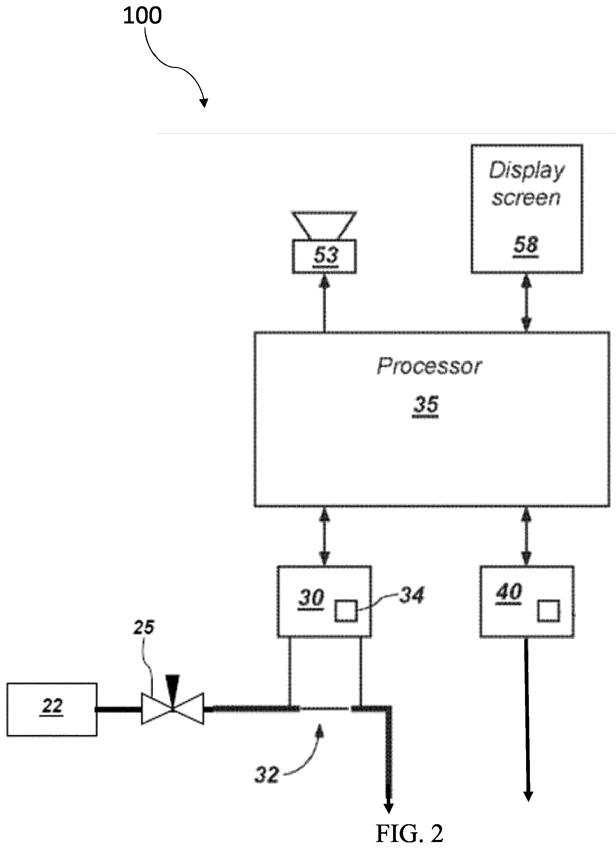

For example, FIG. 2 schematically illustrates one embodiment of a system 100 for monitoring a sedated patient and calibrating the nasal pressure waveforms for the sedated patient, where such calibration will also account for the supplemental oxygen flow being delivered to the patient. Specifically, the system 100 can apply a patient-specific calibration in real-time to the nasal/mask pressure or nasal flow waveform such that the waveform is scaled with a proportional calibration factor to account for anatomical differences and for oxygen-flow-induced positive pressure that make nasal pressure monitoring difficult. The system 100 may include at least one processor 35, a display 58, one or more sensors, an oxygen flow analyzer 30, and an auditory output device 53 (e.g., a speaker). The one or more sensors may include at least a pressure sensor 40. In some embodiments, the system 100 further includes a pressure sensor processor 45 and an oxygen flow analyzer processer 34, each of which may be in communication with the at least one processor 35.

The at least one processor 35 may be a master processor 35 that may be programmed to: receive one or more first respiratory signals corresponding to a nasal pressure of a patient; calculate a calibration factor unique to the patient; actuate a valve (e.g., valve 25) in response to received one or more signals corresponding to a flow of oxygen to the patient; and synthesize a second respiratory signal based on the one or more first respiratory signals, the flow of oxygen to the patient, and the calibration factor. The master processor 35 may receive the one or more first respiratory signals from the pressure sensor 40 and/or the pressure sensor processor 45. The master processor 35 may receive the one or more signals corresponding to a flow of oxygen to the patient from the oxygen flow analyzer 30 and/or the oxygen flow analyzer processer 34.

The display 58 may be for displaying the one or more first respiratory signals corresponding to a nasal pressure of a patient; the synthesized second respiratory signal corresponding to the first respiratory signals, the flow of oxygen to the patient, and the calibration factor; a first visual indicator corresponding to the flow of oxygen to the patient; and a second visual indicator corresponding to an obstruction in an airway of the patient.

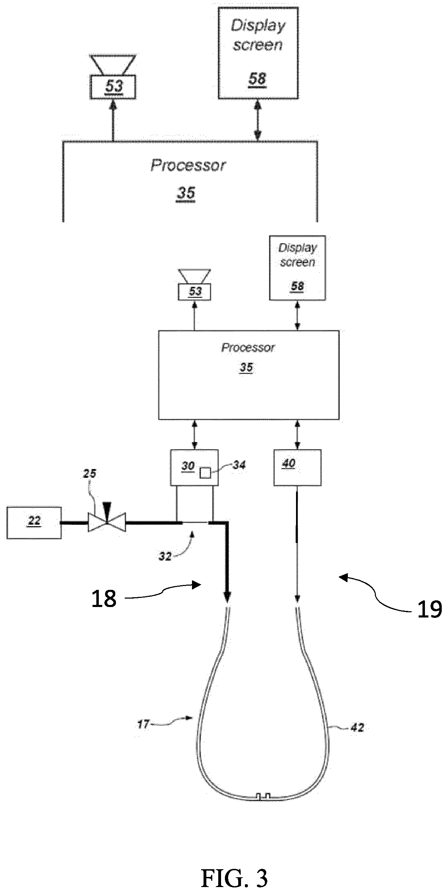

In some embodiments, such as illustrated in FIG. 3, the system 100 may further include an oxygen delivery device 17, which may be a nasal cannula or oxygen mask with an oxygen inlet tube 18 and a separate pressure-sensing tube 19. The pressure sensor 40 may be disposed within or otherwise associated with the pressure-sensing tube. The oxygen inlet tube is connected to a valve (e.g., valve 25) that is actuated by the processor 35 to control oxygen flow from a high-pressure oxygen source. The flow control valve 25 may be a simple needle valve. The flow control valve may be a simple ON/OFF type valve that has only two positions: open and closed. In one configuration, the flow control valve 25 may provide a variable flow. The flow control valve 25 may be connected to the processor 35 and may deliver a variable gas flow. For example the processor may set a flow rate that changes up to 100 times per second according to variables such as the desired oxygen delivery for the particular patient and the measure oxygen delivery to the patient. The variable-type of flow control valve 25 may be, for example, those known in the art such as the EVP Series Proportional Control Valves manufactured by Clippard Instrument Laboratory, Inc.

In some configurations, a flow sensor or differential pressure sensor (e.g., the oxygen flow analyzer 30) is also used in line with the oxygen inlet tube 18 to sense the oxygen flow through the valve 25 that is being administered to the patient. The oxygen flow analyzer 30 measures an oxygen flow rate to provide an oxygen flow rate value. The oxygen flow analyzer 30 may be of any type known in the art, and in one configuration may be a simple differential pressure system. For example, a slight obstruction, such as a section of tubing having a smaller inner diameter 32, is placed in the oxygen flow tubing within the system, and a differential pressure analyzer measures the pressure difference caused by the flow interruption. The measured differential pressure is then used to calculate the corresponding oxygen flow rate. Another type of suitable oxygen flow analyzer 30 could be a heated wire-type anemometer. It may be desirable to include a barometer in the system and compensate for the change in gas density and various altitudes.

The oxygen delivery device 17 is provided with a connection, such as tubing, to an oxygen source 22. It may be desirable to provide a flow control valve 25 in connection with the oxygen source 22, so practitioners can select, adjust, or control the oxygen flow rate from the oxygen source 22. In some embodiments, the flow control valve 25 may be actuated by the processor 35. The flow control valve 25 may be a simple needle valve. The flow control valve 25 may be a simple ON/OFF type valve that has only two positions: open and closed. In one configuration, the flow control valve 25 may provide a variable flow. The flow control valve 25 may be connected to the processor 35 and may deliver a variable gas flow. For example the processor 35 may set a flow rate that changes up to 100 times per second according to variables such as the desired oxygen delivery for the particular patient and the measure oxygen delivery to the patient. The variable-type of flow control valve 25 may be, for example, those known in the art such as the EVP Series Proportional Control Valves manufactured by Clippard Instrument Laboratory, Inc.

Additionally, at some point in the tubing between the patient and the oxygen source 22, an oxygen flow analyzer 30 may be provided. The oxygen flow analyzer 30 measures an oxygen flow rate to provide an oxygen flow rate value. The oxygen flow analyzer 30 may be of any type known in the art, and in one configuration may be a simple differential pressure system. For example, a slight obstruction, such as a section of tubing having a smaller inner diameter, is placed in the oxygen flow tubing within the system 100, and a differential pressure analyzer measures the pressure difference caused by the flow interruption. The measured differential pressure is then used to calculate the corresponding oxygen flow rate. Another type of suitable oxygen flow analyzer 30 could be a heated wire-type anemometer. It may be desirable to include a barometer in the system and compensate for the change in gas density and various altitudes.

The oxygen flow analyzer 30 may be equipped with its own oxygen flow analyzer processor 34 to calculate the oxygen flow rate value from the measured parameters. The oxygen flow analyzer processor 34 may then communicate the oxygen flow rate value to the processor 35. Alternatively, the oxygen flow analyzer 30 may be in communication with the processor 35 to provide the processor 35 with the measured parameters necessary for the processor 35 to calculate the oxygen flow rate value. The processor 35 may be programmed to store and analyze the oxygen flow rate values received from the oxygen flow analyzer processor 34 or the oxygen flow rate values calculated by the processor 35.

The system 100 also includes the pressure sensor 40. The pressure sensor 40 may be a precision, low-pressure sensor. The pressure sensor 40 is in communication with the patient such that the pressure sensor 40 detects a patient's breathing pressure. For example, if the patient is using a mask as an oxygen delivery device 17, the pressure sensor 40 may be in communication with the mask by tubing 42. In the case of a nasal cannula as the oxygen delivery device 17, a piece of tubing 42 in communication with the pressure sensor 40 may be placed in one (or both) of the patient's nostrils. In one configuration, the pressure sensor 40 is provided with a port that allows simple insertion of a piece of tubing 42 (FIG. 3). The tubing 42 can then be placed either on the patient's mask or in a patient's nostril(s). Additionally, the pressure sensor 40 may be disposed anywhere along the tubing it is desired. For example, the pressure sensor 40 may be placed very close to a patient's oxygen delivery device 17, or the pressure sensor 40 may be placed farther from the patient and closer to the medical personnel and other medical instruments.

The pressure sensor 40 detects a patient's breathing pressure (e.g., nasal pressure), including the inhalations, which are detected as a negative pressure, and the exhalations, which are detected as a positive pressure. The plurality of breathing pressure values measured by the pressure sensor 40 may be analyzed by a pressure sensor processor 45 and communicated to the processor 35. Alternatively, the plurality of breathing pressure values measured by the pressure sensor 40 may be communicated to the processor 35 and the processor 35 may analyze the breathing pressure values. The pressure sensor also detects pressures due to oxygen flow.

The processor 35 may receive data from the oxygen flow analyzer 30 (or from the oxygen flow analyzer processor 34) and the pressure sensor 40 (or the pressure sensor processor 45). The processor 35 may be provided with one or more input ports to receive data, and one or more output ports. The processor 35 may be programmed to output an obstruction alarm, an insufficient oxygen flow alarm, or an oxygen delivery device displacement alarm. These alarms may be, for example, audible alarms, visual alarms, or both. The processor 35 may output, for example, to a speaker 53 and/or a visual indicator via the display 58. In other configurations, a separate audible alarm is not provided, but rather hear by the clinician as a series of audible, high-flow oxygen pulses as described in more detail below.

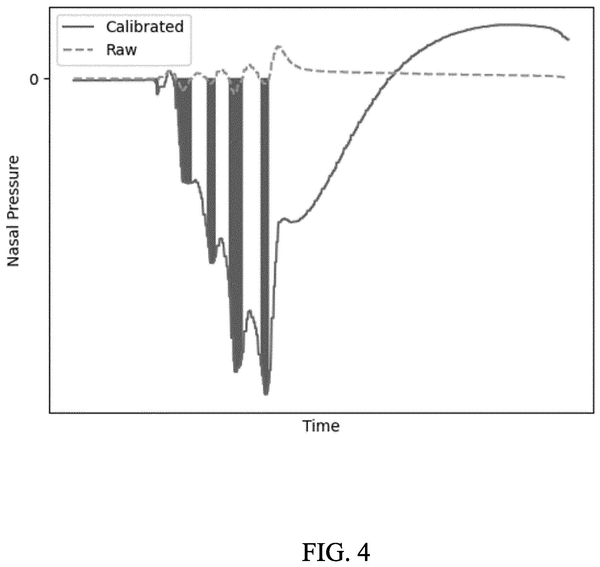

The processor 35 analyzes that data it receives to determine the breathing pattern of the patient and to synthesize a calibrated nasal pressure waveform signal (see FIG. 4) for the patient. The system 100 or the processor 35 may be programmed to periodically administer "test pulses" of oxygen between patient breaths, which includes a brief pulse of high-flow oxygen. The oxygen pulse is delivered during the end expiratory pause of the patient's breathing pattern, which is determined by the processor 35 through signals received from the pressure sensor 40. When the processor 35 determines the patient has finished a breath and there is an end expiratory pause, the processor 35 can send a "test pulse" of high flow of oxygen (i.e., over 10L/min, over 20L/min, over 30L/min, etc.) for a short duration (such as 50 milliseconds).

During this high-flow oxygen pulse, the maximum and minimum nasal pressure and the peak oxygen flow are measured by pressure sensor 40. The peak nasal pressure and peak oxygen flow can be used to establish a pressure-flow relationship in the nose or in the mask for the specific patient. A calibration factor or scale factor is then calculated using the ratio of the flow to the pressure or the square of the pressure. Multiplying the nasal pressure signal by this scale factor produces the scaled nasal pressure signal such that the signal is "normalized" for a given patient, preventing anatomical differences from reducing the signal to an unusable amplitude. Applying this scale factor also synthetically produces the negative portion of the signal that would otherwise be reduced by oxygen flow-induced positive pressure. Adding this signal to the negative of the delivered oxygen flow signal produces a signal proportional to nasal flow that is thus not reduced by anatomical differences or flow-induced positive pressure.

The calibration factor may be calculated from the high-flow oxygen test pulse flow rate and the pressure difference or pressure difference to a power (e.g., to the second power, etc.). For example, the calibration factor may be calculated using Equation 1:

$$K = \frac{Qmax}{(Pmax - Pmin)^2}$$

where K is the scale factor, $Q_{max}$ is a measured flow rate of the pulse of high-flow oxygen, $P_{max}$ is the peak nasal pressure, and $P_{min}$ is the minimum nasal pressure. In other configurations, the calibration factor may be calculated using a variation of Equation 1, Equation 1a:

$$K = \frac{Qmax}{(Pmax - Pmin)^x}$$

In this variation, the difference between $P_{max}$ and $P_{min}$ taken to a power "x" as desired. For example, the power may be 1, 2, 3, 4, etc. The power that the difference is taken will affect the units of the calibration factor. The calibrated signal may be synthesized by the processor 35 from Equation 2:

$$Y = -Q + K * P$$

where Y is the second respiratory signal, K is the scale factor, -Q is the negative of the pulse of high-flow oxygen, and P is the additional nasal pressure measurements.

For example, FIG. 4 illustrates a respiratory signal that has not been normalized ("raw") and a respiratory signal that has been normalized or calibrated ("calibrated") with the scale factor described herein, for a specific sedated patient. This normalized signal can be used for detecting the patient's breathing and, thus, for calculating a respiratory rate. When the signal has been normalized using the scale factor obtained from the high-flow oxygen test pulse, then a fixed threshold value can be applied to the normalized signal to calculate breath rate. This can be done when oxygen is delivered continuously or when it is delivered only while the patient inhales by comparing the signal to a threshold offset from the baseline signal level between breaths, and measuring the time between breaths to calculate the average breathing frequency. When oxygen is delivered only while the patient inhales, the fixed threshold can be relative to zero since there is no oxygen flow or flow-induced pressure until a breath is taken. When oxygen is delivered continuously, in one embodiment the baseline signal is be obtained by averaging the signal between breaths when there are not fluctuations in flow or pressure.

The following is a first example of calculating the calibration factor K for an exemplary patient A. Patient A is being given supplemental oxygen through a nasal cannula. Patient A is breathing at approximately 12 breaths per minute. The system 100 determines Patient A's end expiratory pause and delivers a pulse of high-flow oxygen that is about 50 milliseconds in duration. The measured flow rate, or the maximum measured flow rate, during the pulse, as measured by the oxygen flow analyzer 30, was 26.8 L/min. The peak pressure, as measured by the pressure sensor 40, was 0.62 $cmH_2O$ and the minimum pressure measured was -0.04 $cmH_2O$. The calibration factor K is calculated as the measured flow divided by the difference between the maximum and minimum pressure squared:

$$K = \frac{Qmax}{(Pmax - Pmin)^2}$$

To find the K factor for Patient A, the measured values are plugged into the equation:

$$K = 26.8/(0.62 - 0.04)^2 = 61.52 \; L/min/cmH_2O^2$$

Thus the compensated nasal pressure signal will be -Q + 61.52*P where Q is the oxygen flow given and P is the nasal pressure sensor value.

As another exemplary calculation, suppose Patient A is now being given supplemental oxygen through a non-rebreather mask. The patient is still breathing approximately 12 breaths per minute. Between the breaths, the system 100 applies the same high-flow oxygen pulse that is 50 milliseconds in duration. The maximum flow rate during the high-flow pulse, as measured by the oxygen flow analyzer 30, is now 27.1 L/min. The peak pressure, as measured by pressure sensor 40, was much lower because the pressure generated by a pulse into a mask is much smaller than a pulse into the nostrils, and was measured to be 0.26 $cmH_2O$. The minimum pressure measured was 0.01 $cmH_2O$. The calibration factor K can be calculated as in the example above.

$$K = 27.1/(0.26 - 0.01)^2 = 433.6 \; L/min/cmH_2O^2$$

Thus the compensated nasal pressure signal will be -Q + 433.6*P.

Note that in both calculation examples, the pressure difference was squared. This results in a calibration factor K that has units of $L/min/cmH_2O^2$, which when multiplied by the pressure P, gives a unit of $L/min/cmH_2O$. The same calculation could be done without squaring the pressure to produce a slightly different K factor with units of $L/min/ cmH_2O$, which when multiplied by the pressure, gives units of L/min.

The use of the calibration factor create a signal that is proportional to flow through the nose/mouth. The current equation assumes a linear pressure-flow relationship, and characterizes the relationship based on a single data point $(Q_{max}, P_{max} - P_{min})$. In reality, the relationship is not exactly linear, which is why squaring the pressure difference in the denominator may result in a signal that more accurately resembles the flow through the nose/mouth. This allows the calculation to be made quickly and in real-time while applying only a single test pulse, which can be done between breaths. The signal may not be quantitatively accurate, but it qualitatively shows what is happening physiologically for nasal pressure and flow, which is difficult to do with the raw nasal pressure signal since it is disrupted by oxygen flow.

According to another aspect of the disclosure, methods for determining airway obstruction are disclosed. It is also common for the drugs used during sedation to relax the muscles that normally hold open the upper airway, causing the airway tissues to partially or completely occlude the flow of air in and out of the lungs. This relaxation effect frequently results in patients that have an open airway during exhalation, but an obstructed airway during inhalation. The negative pressure created as the patient tries to inhale may further close the airway, exacerbating the problem. Airway obstruction of this type can sometimes present itself with audible snoring, but at other times it is much more difficult to identify. Clinicians can sometimes help sedated patients overcome these obstructions by lifting their chin. However, if the obstruction cannot be identified, the clinician does not know this type of action is needed, and there is an increased risk for inadequate oxygen intake to the patient's lungs.

When delivering oxygen only while the patient inhales, airway obstruction can be visually identified using the calibrated nasal pressure signal by adding a visual change on a display of the signal, such as color. Using the same system described previously, the system algorithm reads the nasal pressure signal for fluctuations in negative pressure. High flow oxygen pulses are delivered synchronized with these fluctuations by actuating the flow control valve 25. When the airway is obstructed, the oxygen pulses will create a positive pressure and the system discontinue oxygen flow in response until a negative pressure is observed again. When the airway is momentarily open and oxygen pulses are being given, the signal is displayed in one color, and when oxygen is not given, the signal is displayed in another color.

This creates a visual waveform with an alternating visual pattern that can be used to visually identify airway obstruction, as illustrated by the dark bands in FIG. 4. While the oxygen is flowing, the majority of the signal is derived from oxygen flow (-Q in Equation 1) and while the oxygen is off, the signal is primarily calculated as the pressure scaled by calibration factor K ($K * P$ in Equation 1). The alternating color in the plot, with the dark bands indicating an oxygen pulse and white spaces indicating no oxygen given, provides an identifiable visual pattern associated with airway obstruction. The system 100 also produces an auditory means of identifying airway obstruction that corresponds with the visual means. Oxygen pulses are intentionally given at high flows (e.g., about 30 liters-per-minute (L/min)) such that each oxygen pulse delivered by the system 100 creates an audible sound from the high flow of oxygen. The nozzle in the oxygen connector creates an audible sound in proportion to the oxygen flow rate.

As the high flow is turned on and off by the system 100 during inhalation, it creates an audio signature that helps the practitioner to understand the breathing pattern of the patient by listening to the audio signature. For example, the staccato on/off nature of this sound is indicative of intermittent airway obstruction, long pauses between noises indicate slow breathing, etc., By applying the color and/or visual change to the calibrated nasal pressure signal whenever oxygen flow is given at a flow rate that produces an audible sound, the combined auditory and visual cues provide a multi-sensory means for clinicians to identify airway obstruction and other respiratory conditions.

Other examples of systems capable of monitoring a patient's breathing while sedated are illustrated and described in U.S. Patent No. 10,159,815 issued on December 25, 2018 and entitled "SYSTEM AND METHOD FOR DETECTION OF OXYGEN DELIVERY FAILURE," the entire contents of which are herein incorporated by reference.

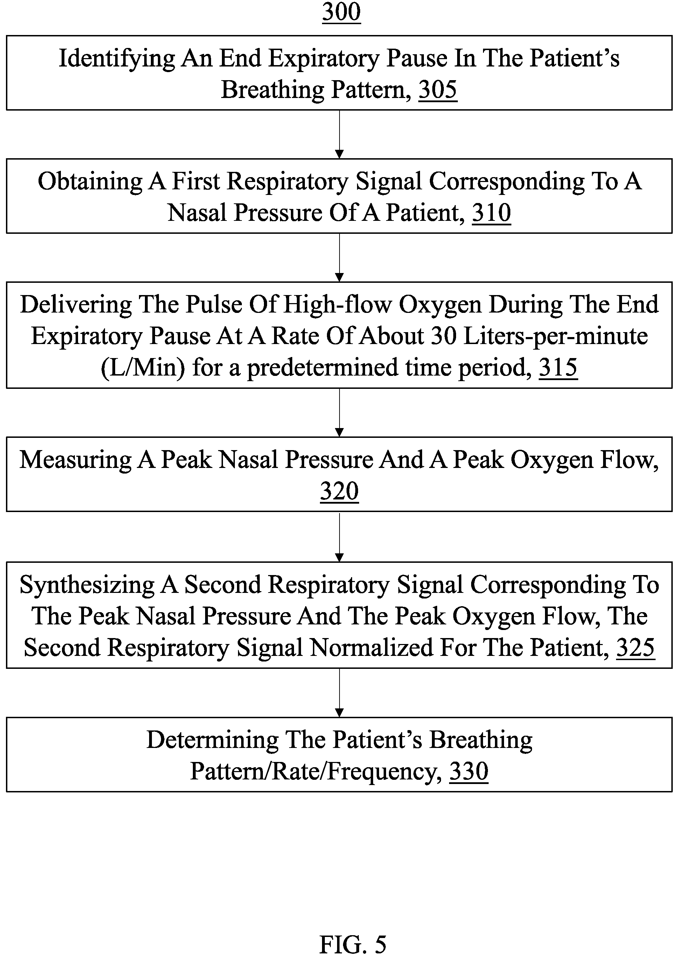

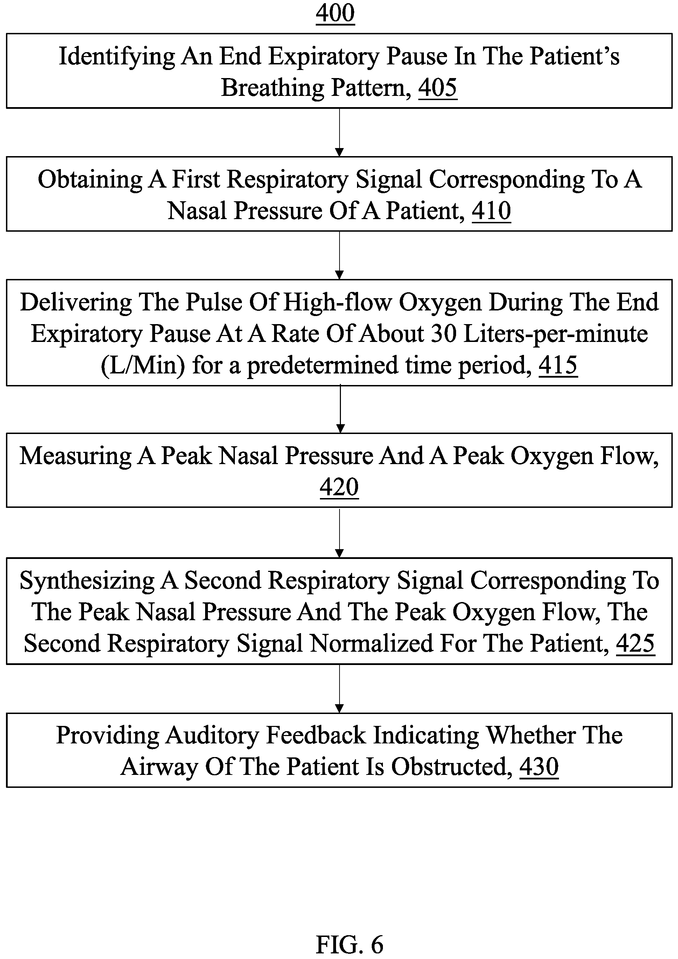

FIGS. 5 and 6 are flowcharts of methods of monitoring a sedated patient. Though the flowcharts are illustrated with the steps in a certain order, the steps of the methods may be taken in a different order or any appropriate order. Additionally, more than one step may be taken at a time or some steps may be taken synchronously and other steps are taken asynchronously. FIG. 5 is a flowchart of a method 300 of monitoring a patient's breathing while sedated. The method 300 may include identifying an end expiratory pause in the patient's breathing pattern, at 305. The method 300 may also include obtaining a first respiratory signal corresponding to a raw or uncalibrated nasal pressure of a patient, at 310. Additionally, the method 300 may include delivering a pulse of high-flow oxygen during the end expiratory pause at a rate of about 30 liters-per-minute (L/min) for a predetermined amount of time (such as less than 0.5 seconds, less than 0.1 seconds, less than 0.01 seconds, etc.), at 315. Further, the method 300 may include measuring a peak nasal pressure and a peak oxygen flow, at 320. Still further, the method 300 may include synthesizing a second respiratory signal corresponding to the peak nasal pressure and the peak oxygen flow, the second respiratory signal normalized for the patient, at 325. The method 300 may also include determining the patient's breathing pattern, rate, or frequency, at 330.

Obtaining a first respiratory signal corresponding to a nasal pressure of a patient may include measuring the nasal pressure of the patient over time and plotting the nasal pressure over time, the first respiratory signal being unique to the patient. Synthesizing a second respiratory signal corresponding to the peak nasal pressure and the peak oxygen flow may include calculating a scale factor from the pulse of high-flow oxygen, a pressure difference between the peak nasal pressure, and a minimum nasal pressure. Synthesizing the second respiratory signal may also include scaling the first respiratory signal with the scale factor to account for the patient's anatomy and establishing a pressure-flow relationship from the peak nasal pressure and the peak oxygen flow. Synthesizing the second respiratory signal may further include multiplying additional nasal pressure measurements by the scale factor K, adding scaled additional nasal pressure measurements to a negative of the pulse of high-flow oxygen, and obtaining the second respiratory signal.

The scale or calibration factor K may be calculated using Equation 1:

$$K = \frac{Qmax}{(Pmax - Pmin)^2}$$

The synthesized second respiratory signal may be a compensated respiratory signal, accounting for the patient's breathing pattern and the pulse of high-flow oxygen.

FIG. 6 is a flowchart of a method 400 of determining whether an airway of a sedated patient is obstructed. The method 400 may include identifying an end expiratory pause in the patient's breathing pattern, at 405. The method 400 may also include obtaining a first respiratory signal corresponding to a nasal pressure of a patient, at 410. Additionally, the method 400 may include delivering a pulse of high-flow oxygen during the end expiratory pause at a rate of about 30 liters-per-minute (L/min) for a predetermined time period (such as less than 0.5 seconds, less than 0.05 seconds, etc.) at 415. Further, the method 400 may include measuring a peak nasal pressure and a peak oxygen flow, at 420. Still further, the method 400 may include synthesizing a second respiratory signal corresponding to the peak nasal pressure and the peak oxygen flow, the second respiratory signal normalized for the patient, at 425. The method 400 may also include providing auditory feedback indicating whether the airway of the patient is obstructed, at 430.

In some embodiments, the method 400 may additionally include displaying a visual indicator that the airway of the patient is obstructed. Displaying a visual indicator may include overlaying a color on the second respiratory signal for the patient, the second respiratory signal being a calibrated nasal pressure signal for the patient. Additionally, and/or alternatively, displaying a visual indicator may include displaying the second respiratory signal in a first color corresponding to a positive pressure and displaying the second respiratory signal in a second color corresponding to a negative pressure.

Administering a pulse of high-flow oxygen may include flowing the pulse of high-flow oxygen through a cannula, thereby providing auditory feedback indicating whether the airway of the patient is obstructed.

*Additional Terms and Definitions*

While particular embodiments have been illustrated and described herein, it should be understood that various other changes and modifications may be made without departing from the spirit and scope of the claimed subject matter. Moreover, although various aspects of the claimed subject matter have been described herein, such aspects need not be utilized in combination.

In one embodiment, the terms "about" and "approximately" refer to numerical parameters within 10% of the indicated range. The terms "a," "an," "the," and similar referents used in the context of describing the embodiments of the present disclosure (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the embodiments of the present disclosure and does not pose a limitation on the scope of the present disclosure. No language in the specification should be construed as indicating any non-claimed element essential to the practice of the embodiments of the present disclosure.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the author(s) of this disclosure for carrying out the embodiments disclosed herein. Of course, variations on these described embodiments will become apparent to those of ordinary skill in the art upon reading the foregoing description. The author(s) expects skilled artisans to employ such variations as appropriate, and the author(s) intends for the embodiments of the present disclosure to be practiced otherwise than specifically described herein. Accordingly, this disclosure includes all modifications and equivalents of the subject matter recited in the claims appended hereto as permitted by applicable law. Moreover, any combination of the above-described elements in all possible variations thereof is encompassed by the present disclosure unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of this disclosure so claimed are inherently or expressly described and enabled herein.

Although this disclosure provides many specifics, these should not be construed as limiting the scope of any of the claims that follow, but merely as providing illustrations of some embodiments of elements and features of the disclosed subject matter. Other embodiments of the disclosed subject matter, and of their elements and features, may be devised which do not depart from the spirit or scope of any of the claims. Features from different embodiments may be employed in combination. Accordingly, the scope of each claim is limited only by its plain language and the legal equivalents thereto.

The invention refers also to a method of monitoring a patient's respiration while sedated, the method comprising: identifying an end expiratory pause in the patient's breathing pattern; obtaining a first respiratory signal corresponding to a nasal pressure of a patient; delivering a pulse of high-flow oxygen during the end expiratory pause at a rate of about 30 liters-per-minute (L/min) and for a predetermined time period; measuring the oxygen flow rate during the pulse of high-flow oxygen; measuring a peak nasal pressure and a peak oxygen flow during the pulse of high-flow oxygen; synthesizing a second respiratory signal corresponding to the peak nasal pressure and the peak oxygen flow, the second respiratory signal normalized for the patient; and determining the patient's respiration.

Preferred is the method, wherein obtaining a first respiratory signal corresponding to a nasal pressure of a patient comprises: measuring the nasal pressure of the patient over time; and plotting the nasal pressure over time, the first respiratory signal being unique to the patient.

Preferred is the method, wherein synthesizing a second respiratory signal corresponding to the peak nasal pressure and the peak oxygen flow comprises: calculating a scale factor from the oxygen flow rate during the pulse of high-flow oxygen, and pressure difference between the peak nasal pressure and a minimum nasal pressure during the pulse of high-flow oxygen; scaling the first respiratory signal with the scale factor to account for the patient's anatomy; and establishing a pressure-flow relationship from the peak nasal pressure and the peak oxygen flow.

Preferred is the method, wherein the scale factor is calculated as:

$$K = \frac{Qmax}{(Pmax - Pmin)^{\mathbf{x}}}$$

wherein K is the scale factor, $Q_{max}$ is a measured flow rate of the pulse of high-flow oxygen, $P_{max}$ is the peak nasal pressure, and $P_{min}$ is the minimum nasal pressure.

Preferred is the method, wherein synthesizing a second respiratory signal corresponding to the peak nasal pressure and the peak oxygen flow further comprises: multiplying additional nasal pressure measurements by the scale factor K; adding scaled additional nasal pressure measurements to a negative of the pulse of high-flow oxygen; and obtaining the second respiratory signal.

Preferred is the method, wherein the second respiratory signal is calculated as follows:

$$Y = -Q + K * P$$

wherein Y is the second respiratory signal, K is the scale factor, -Q is the negative of the pulse of high-flow oxygen, and P is the additional nasal pressure measurements.

Preferred is the method, wherein the second respiratory signal is a compensated respiratory signal, accounting for the patient's breathing pattern and the pulse of high-flow oxygen.

Preferred is the method, wherein Y is utilized to calculate a respiratory rate for the patient.

The invention refers also to a method of determining whether an airway of a sedated patient is obstructed, the method comprising: identifying an end expiratory pause in the patient's breathing pattern; obtaining a first respiratory signal corresponding to a nasal pressure of a patient; delivering a pulse of high-flow oxygen during the end expiratory pause at a rate of about 30 liters-per-minute (L/min) for a predetermined time period; measuring a peak nasal pressure and a peak oxygen flow; synthesizing a second respiratory signal corresponding to the peak nasal pressure and the peak oxygen flow, the second respiratory signal normalized for the patient; and providing auditory feedback indicating whether the airway of the patient is obstructed.

Preferred is the method, further comprising displaying a visual indicator that the airway of the patient is obstructed.

Preferred is the method, wherein displaying a visual indicator comprises overlaying a color on the second respiratory signal for the patient, the second respiratory signal being a calibrated nasal pressure signal for the patient.

Preferred is the method, wherein displaying a visual indicator comprises displaying the second respiratory signal in a first color corresponding to a positive pressure and displaying the second respiratory signal in a second color corresponding to a negative pressure.

Preferred is the method, wherein the predetermined time period is less than about 0.1 seconds and wherein administering the pulse of high-flow oxygen comprises flowing the pulse of high-flow oxygen through a cannula, the high-flow oxygen through the cannula creating the auditory feedback.

The invention refers also to a system for applying a patient-specific calibration in real-time to a nasal/mask pressure or nasal flow waveform, the system comprising: a nasal cannula having an oxygen inlet tube and a pressure-sensing tube separate from the oxygen inlet tube; a processor-actuated valve for controlling oxygen flow from a high-pressure oxygen source to the nasal cannula; a flow sensor in connection with the oxygen inlet tube and for monitoring a flow of oxygen through the processor-actuated valve; a pressure sensor in connection with the pressure-sensing tube; and a processor for receiving one or more pressure signals from the pressure sensor, the processor in communication with the processor-actuated valve, the processor programmed to: receive one or more first respiratory signals corresponding to a nasal pressure of a patient; actuate the processor-actuated valve upon determination of an end expiratory pause in a breathing pattern of the patient to deliver a high-flow pulse of oxygen of at least 25 L/min to a patient; receive a maximum and minimum nasal pressure from the pressure sensor; receive one or more signals corresponding to a flow of oxygen to the patient from the flow sensor; calculate a calibration factor unique to the patient; and synthesize a second respiratory signal based on the one or more first respiratory signals, the flow of oxygen to the patient, and the calibration factor.

Preferred is the system, wherein the calibration factor is calculated as:

$$K = \frac{Qmax}{(Pmax - Pmin)^2}$$

wherein K is the scale factor, $Q_{max}$ is a measured flow rate of the pulse of high-flow oxygen, $P_{max}$ is the maximum nasal pressure, and $P_{min}$ is the minimum nasal pressure.

Preferred is the system, further comprising a display in association with the processor, the display for providing visual indications correlated to a nasal pressure signal of the patient.

Preferred is the system, wherein actuation of the processor-actuated valve causes delivery of a pulse of high-flow oxygen to the patient to be inhaled by the patient after the end expiratory pause.

Preferred is the system, wherein the pulse of high-flow oxygen comprises delivery of oxygen at a rate of about 30 L/min for a time period of about 0.5 seconds.

The invention refers also to a system comprising: a processor programmed to: receive one or more first respiratory signals corresponding to a nasal pressure of a patient, calculate a calibration factor unique to the patient, receive one or more signals corresponding to a flow of oxygen to the patient, and synthesize a second respiratory signal based on the one or more first respiratory signals, the flow of oxygen to the patient, and the calibration factor; and a display in communication with the processor, the display for displaying: the one or more first respiratory signals corresponding to a nasal pressure of a patient, the synthesized second respiratory signal corresponding to the first respiratory signals, the flow of oxygen to the patient, and the calibration factor, a first visual indicator corresponding to the flow of oxygen to the patient, and a second visual indicator corresponding to an obstruction in an airway of the patient.

Preferred is the system, wherein the processor is further programmed to actuate a valve in response to received one or more signals corresponding to a flow of oxygen to the patient.

Preferred is the system having at least one preferred feature outlined above, especially outlined for the method or the system outlined above.

**Claims**

1. A system comprising:
   a processor (35) programmed to:

   receive one or more first respiratory signals corresponding to a nasal pressure of a patient,
   calculate a calibration factor unique to the patient,
   receive one or more signals corresponding to a flow of oxygen to the patient, and
   synthesize a second respiratory signal based on the one or more first respiratory signals, the flow of oxygen to the patient, and the calibration factor; and
   a display (58) in communication with the processor (35), the display (58) for displaying:

   the one or more first respiratory signals corresponding to a nasal pressure of a patient, the synthesized second respiratory signal corresponding to the first respiratory signals, the flow of oxygen to the patient, and the calibration factor,
   a first visual indicator corresponding to the flow of oxygen to the patient, and
   a second visual indicator corresponding to an obstruction in an airway of the patient.

2. The system of claim 1, wherein the processor (35) is further programmed to actuate a processor-actuated valve (25) in response to received one or more signals corresponding to a flow of oxygen to the patient.

3. The system of claim 1 or claim 2, further comprising:

   an oxygen delivery device (17) having an oxygen inlet tube (18) and a pressure-sensing tube (19) separate from the oxygen inlet tube (18);
   the processor-actuated valve (25) for controlling oxygen flow from a high-pressure oxygen source to the oxygen delivery device (17);
   a flow sensor (30) in connection with the oxygen inlet tube (18) and for monitoring a flow of oxygen through the processor-actuated valve (25);
   a pressure sensor (40) in connection with the pressure-sensing tube (19); and

EP 4 717 162 A1

the processor (35) for receiving one or more pressure signals from the pressure sensor (40), the processor (35) in communication with the processor-actuated valve (25).

4. The system of any one of claims 1-3, wherein the processor (35) is programmed to actuate the processor-actuated valve (25) upon determination of an end expiratory pause in a breathing pattern of the patient to deliver a high-flow pulse of oxygen of at least 25 L/min to a patient.

5. The system of any one of claims 1-4, wherein the processor (35) is programmed receive a maximum and minimum nasal pressure from the pressure sensor (40).

6. The system of claims 1-4, wherein the calibration factor is calculated as:

$$K = \frac{Qmax}{(Pmax - Pmin)^{\mathbf{x}}}$$

wherein K is the scale factor, Qmax is a measured flow rate of the pulse of high-flow oxygen, Pmax is the maximum nasal pressure, and Pmin is the minimum nasal pressure.

7. The system of claims 1-4, wherein actuation of the processor-actuated valve (25) causes delivery of the pulse of high-flow oxygen to the patient to be inhaled by the patient after the end expiratory pause.

8. The system of claim 7, wherein the pulse of high-flow oxygen comprises delivery of oxygen at a rate of about 30 L/min for a time period of about 0.5 seconds.

9. The system of any one of claims 1-8, wherein the processor (35) is programmed to determine when the patient has finished a breath and there is an end expiratory pause.

10. The system of any one of claims 1-9, wherein receiving a first respiratory signal corresponding to a nasal pressure of a patient comprises the processor (35):

    measuring the nasal pressure of the patient over time; and
    plotting the nasal pressure over time, the first respiratory signal being unique to the patient.

11. The system of any one of claims 1-10, wherein the processor (35) synthesizing a second respiratory signal corresponding to the peak nasal pressure and the peak oxygen flow comprises:

    calculating a scale factor from the oxygen flow rate during the pulse of high-flow oxygen, and pressure difference between the peak nasal pressure and a minimum nasal pressure during the pulse of high-flow oxygen;
    scaling the first respiratory signal with the scale factor to account for the patient's anatomy; and
    establishing a pressure-flow relationship from the peak nasal pressure and the peak oxygen flow.

12. The system of any one of claims 1-11, wherein the processor (35) synthesizing a second respiratory signal corresponding to the peak nasal pressure and the peak oxygen flow further comprises:

    multiplying additional nasal pressure measurements by the scale factor K;
    adding scaled additional nasal pressure measurements to a negative of the pulse of high-flow oxygen; and
    obtaining the second respiratory signal.

13. The system of claim 12, wherein the second respiratory signal is calculated as follows:

$$Y = -Q + K * P$$

wherein Y is the second respiratory signal, K is the scale factor, -Q is the negative of the pulse of high-flow oxygen, and P is the additional nasal pressure measurements.

14. The system of claims 12-13, wherein the second respiratory signal is a compensated respiratory signal, accounting for the patient's breathing pattern and the pulse of high-flow oxygen.

15. The system of claim 13 or claim 14, wherein Y is utilized to calculate a respiratory rate for the patient.

FIG. 1A

FIG. 1B

100

Display
screen

58

53

Processor

35

30    34

40

25

22

32

FIG. 2

FIG. 3

FIG. 4

<u>300</u>

```
┌─────────────────────────────────────────────────────────────┐
│  Identifying An End Expiratory Pause In The Patient's         │
│              Breathing Pattern, 305                           │
└─────────────────────────────────────────────────────────────┘
                              │
                              ▼
┌─────────────────────────────────────────────────────────────┐
│  Obtaining A First Respiratory Signal Corresponding To A      │
│            Nasal Pressure Of A Patient, 310                   │
└─────────────────────────────────────────────────────────────┘
                              │
                              ▼
┌─────────────────────────────────────────────────────────────┐
│  Delivering The Pulse Of High-flow Oxygen During The End      │
│  Expiratory Pause At A Rate Of About 30 Liters-per-minute     │
│      (L/Min) for a predetermined time period, 315             │
└─────────────────────────────────────────────────────────────┘
                              │
                              ▼
┌─────────────────────────────────────────────────────────────┐
│  Measuring A Peak Nasal Pressure And A Peak Oxygen Flow,      │
│                      320                                      │
└─────────────────────────────────────────────────────────────┘
                              │
                              ▼
┌─────────────────────────────────────────────────────────────┐
│  Synthesizing A Second Respiratory Signal Corresponding To    │
│  The Peak Nasal Pressure And The Peak Oxygen Flow, The        │
│  Second Respiratory Signal Normalized For The Patient, 325    │
└─────────────────────────────────────────────────────────────┘
                              │
                              ▼
┌─────────────────────────────────────────────────────────────┐
│  Determining The Patient's Breathing                          │
│        Pattern/Rate/Frequency, 330                            │
└─────────────────────────────────────────────────────────────┘
```

FIG. 5

400

| Identifying An End Expiratory Pause In The Patient's Breathing Pattern, 405 |
| :---: |

↓

| Obtaining A First Respiratory Signal Corresponding To A Nasal Pressure Of A Patient, 410 |
| :---: |

↓

| Delivering The Pulse Of High-flow Oxygen During The End Expiratory Pause At A Rate Of About 30 Liters-per-minute (L/Min) for a predetermined time period, 415 |
| :---: |

↓

| Measuring A Peak Nasal Pressure And A Peak Oxygen Flow, 420 |
| :---: |

↓

| Synthesizing A Second Respiratory Signal Corresponding To The Peak Nasal Pressure And The Peak Oxygen Flow, The Second Respiratory Signal Normalized For The Patient, 425 |
| :---: |

↓

| Providing Auditory Feedback Indicating Whether The Airway Of The Patient Is Obstructed, 430 |
| :---: |

FIG. 6

Europäisches
Patentamt
European
Patent Office
Office européen
des brevets

# EUROPEAN SEARCH REPORT

**Application Number**

EP 25 20 3449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/166797 A1 (ORR JOSEPH [US])<br>16 June 2016 (2016-06-16)<br>* figures 1-9 *<br>* paragraphs [0084] - [0134] * | 1-15 | INV.<br>A61B5/08<br>A61B5/087<br>A61M16/00<br>A61B5/00 |
| A | EP 2 668 900 A1 (NIHON KOHDEN CORP [JP]; UNIV CHIBA NAT UNIV CORP [JP])<br>4 December 2013 (2013-12-04)<br>* figure 2 *<br>* paragraphs [0017] - [0055] * | 1-15 | |
| A | HEBBINK RUTGER H.J. ET AL: "Tidal spirometric curves obtained from a nasal cannula",<br>MEDICAL ENGINEERING & PHYSICS.,<br>vol. 97, 1 November 2021 (2021-11-01),<br>pages 1-9, XP093333110,<br>GB<br>ISSN: 1350-4533, DOI:<br>10.1016/j.medengphy.2021.09.004<br>* abstract * | 1-15 | |

**TECHNICAL FIELDS SEARCHED (IPC)**

A61B
A61M

The present search report has been drawn up for all claims

| Place of search | Date of completion of the search | Examiner |
|---|---|---|
| The Hague | 18 November 2025 | Athanasiadis, I |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 20 3449

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2025

| Patent document cited in search report | Publication date | Patent family member(s) | | Publication date |
|---|---|---|---|---|
| US 2016166797 A1 | 16-06-2016 | EP | 3229884 A1 | 18-10-2017 |
| | | US | 2016166797 A1 | 16-06-2016 |
| | | WO | 2016094664 A1 | 16-06-2016 |
| EP 2668900 A1 | 04-12-2013 | CN | 103445781 A | 18-12-2013 |
| | | EP | 2668900 A1 | 04-12-2013 |
| | | JP | 6075972 B2 | 08-02-2017 |
| | | JP | 2013248011 A | 12-12-2013 |
| | | US | 2013324877 A1 | 05-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10159815 B